# EUROPÄISCHE PATENTSCHRIFT

(11) **EP 1 871 248 B1**
(45) Veröffentlichungstag und Bekanntmachung des Hinweises auf die Patenterteilung: **18.05.2011**
(21) Anmeldenummer: 06723911.1
(22) Anmeldetag: 28.03.2006
(51) Int. Cl.: A61B 17/64

(54) **FIXATIONSEINRICHTUNG ZUM STABILEN VERBINDEN WENIGSTENS ZWEIER KNOCHENTEILE EINES GEBROCHENEN KNOCHENS SOWIE FIXATIONSELEMENT UND BAUSATZ**
FIXATION DEVICE FOR STABLY INTERLINKING AT LEAST TWO BONE FRAGMENTS OF A BROKEN BONE AND CORRESPONDING FIXATION ELEMENT AND KIT
DISPOSITIF DE FIXATION PERMETTANT LA LIAISON STABLE D'AU MOINS DEUX PARTIES D'UN OS BRISE, ELEMENT DE FIXATION ET KIT

(30) Priorität: 01.04.2005 DE 202005005444 U
(43) Veröffentlichungstag der Anmeldung: 02.01.2008
(73) Patentinhaber: Tantum AG, 24537 Neumünster (DE)
(72) Erfinder: BAUER, Eckhard, 24105 Kiel (DE)
(74) Vertreter: Wenzel & Kalkoff
(86) Internationale Anmeldenummer: PCT/EP2006/002951
(87) Internationale Veröffentlichungsnummer: WO 2006/103087

(56) Entgegenhaltungen:
- US-A- 5 393 161
- US-A1- 2003 109 879

## Beschreibung

Die Erfindung betrifft eine Fixationseinrichtung zum stabilen Verbinden wenigstens zweier Knochenteile eines gebrochenen Knochens, umfassend wenigstens einen Grundkörper zur festen Aufnahme jeweils wenigstens eines Knochennagels, wenigstens eine Fixationsstange, die mittels Klemmverbindung mit dem Grundkörper verbindbar ist, wobei Grundkörper und Fixationsstange in einem ersten Verbindungszustand der Fixationseinrichtung zum Einstellen ihrer relativen Position freigegeben sind und in einem zweiten Verbindungszustand zum Fixieren der eingestellten Position durch Kraftschluss steif miteinander verbunden sind, einen an dem Grundkörper winkelverstellbar gelagerten Klemmhalter, der eine Fixationsstange zum Verändern und Einstellen ihrer Position in einer Aufnahme aufnimmt, ein den Klemmhalter an dem Grundkörper lagerndes Lager und eine Verbindung mit einem Spannstab, an dem zum Gegeneinanderspannen der Grundkörper, der Stangen-Klemmhalter und das Lager für den Klemmhalter in Reihe angeordnet sind, wobei einerseits bei gelöster Spannstabverbindung die Freigabe-Verbindung und andererseits bei ausreichend großer Schließspannung in der Spannstab-verbindung die stabile Kraftschluss-Verbindung hergestellt sind, wobei der Stangen-Klemmhalter durch einen die Fixationsstange sphärisch verstellenden Halter gebildet ist, der in einem Bereich zum Verstellen und Beibehalten der eingestellten Position an dem Grundkörper in alle Raumrichtungen geführt ist, wobei sich der Klemmhalter und wenigstens ein von dem Spannstab durchfasstes Lagerelement des den Klemmhalter mit dem Grundkörper verbindenden Lagers im die räumliche Führung herstellenden Formschluss befinden. Die Erfindung bezieht sich auch auf ein Fixationselement einer solchen Einrichtung und einen ihre Bauteile aufnehmenden Bausatz in steriler Verpackung.

Eine gattungsgemäße Einrichtung, die auch als Fixateur bezeichnet wird, dient dazu, eine Fixationsverbindung zwischen Knochenteilen herzustellen, um Frakturen zu behandeln. Insbesondere werden solche Fixateure bei Radiusfrakturen mit Gelenkbeteiligung angelegt. In die Knochenteile eingebrachte Knochennägel müssen steif mit wenigstens einer Fixationsstange verbunden werden, um das Zusammenwachsen der Knochenteile in richtiger Stellung zueinander zu ermöglichen. Nach Maßgabe der Anordnung und Ausrichtung der Knochennägel sowie der zu überbrückenden Strecke muss die Fixationsstange ausgerichtet werden können. In ausgerichteter Position muss sie steif mit den Grundkörpern verbindbar sein, die ihrerseits in stabiler steifer Verbindung an den Knochennägeln festzusetzen sind.

An die Fixateure wird eine Reihe von Anforderungen gestellt. Es muss große Stabilität gewährleistet werden. Fixierte Knochen dürften während auch mehrwöchiger Tragezeit des Fixateurs ihre Position zueinander nicht verändern. Dies erfordert hohe Stabilität insbesondere in den Verbindungen einzelner Bauteile. Der Fixateur soll einfach handhabbar sein, um die Operationszeit zu verkürzen. Zudem soll er gewichtsmäßig relativ leicht bauen. Die Fixationseinrichtung soll auch kostengünstig zur Verfügung stehen. Dies hat insbesondere im Hinblick auf eine Einmalverwendung besondere Bedeutung, wenn sich diese gegenüber der Aufbereitung der Komponenten zur Wiederverwendung rentiert. So werden Fixateure, die in Folge hoher Anschaffungskosten mehrfach verwendet werden sollten, nach ihrer Anwendung häufig nicht zurückgegeben, so dass dadurch Verlust entsteht. Auch sind bei Wiederverwendung besondere Abläufe und Maßnahmen insbesondere zum Sterilisieren erforderlich.

Bekannte Fixateure werden den genannten Anforderungen nicht gerecht. Fixationselemente eines gattungsgemäßen Fixateurs umfassen einen Grundkörper mit einem Stangen-Klemmlager, das zum Positionieren einer Fixationsstange sphärisch verstellbar ist, wobei Teile des Fixationselements mittels eines Spannstabes verbunden sind (US 5 393 161 A). Eine Klemmhalter-Kugel ist in eine Gelenkverbindung eingekapselt. Infolge dessen baut das Fixationselement sperrig, und die Handhabung ist erschwert. Zum Aufbau einer Fixationseinrichtung sind zusätzlich Schraubklemmen für Knochennägel und Schraubverbindungen für Fixationsstangen erforderlich. Aus US 2003/109879 A1 bekannte Fixationsselemente sind zum Positionieren einer Fixationsstange in einer Schraubverbindung in nur zwei Ebenen verstellbar. Zum Verstellen in weiteren Ebenen sind zusätzliche Teile mit zusätzlichen Schraubverbindungen vorgesehen. Klemmelemente sind an unterschiedlichen Lagerachsen angeordnet. Auch noch andere Klemmhalter-Einrichtungen mit mehreren Schraubverbindungen an unterschiedlichen Lagerachsen sind in gelöstem Zustand relativ schwer handhabbar, sie neigen dazu, sich in gespanntem Zustand zu lösen, und sie werden mit relativ schweren Klemmteilen aus Metall gefertigt. Fixateure gemäß US 2003/109879 A1 und andere bekannte Fixateure sehen für Knochennägel und Fixationsstangen jeweils eine Schraubverbindung vor. Bei diesen Einrichtungen ist die Handhabung besonders erschwert, infolge der Mehrzahl der Schraubverbindungen besteht Anfälligkeit zur Instabilität, und für die Schraubverbindungen werden relativ massive metallische Elemente verwendet, um die Verbindungsfestigkeit zu erhöhen.

Der Erfindung liegt das Ziel zugrunde, eine Fixationseinrichtung derart zu gestalten, dass sie besonders einfach handhabbar ist und mit relativ wenig Bauteilen kostengünstig zur Verfügung steht, wobei die Verbindungssteifigkeit besonders groß und zuverlässig über auch längeren Zeitraum gewährleistet sein soll. Dennoch soll es möglich werden, die Bauteile der Einrichtung aus Leichtwerkstoff und in kostengünstiger Massenproduktion, insbesondere aus im Spritzgussverfahren hergestellten Kunststoffteilen herzustellen. Die Herstellung soll gegebenenfalls so kostengünstig sein, dass das Produkt als billiges Einwegprodukt verwendbar ist, um insbesondere in einer Klinik die Abläufe zu vereinfachen, indem Rückführung, Wiederaufbereitung und Sterilisierung bereits verwendeter Fixateure vermieden werden.

Die Ziele der Erfindung werden in Verbindung mit den Merkmalen der Fixationseinrichtung der eingangs genannten Art dadurch erreicht, dass der Klemmhalter auf dem Spannstab aufgereiht ist, wobei er eine Durchgangsöffnung aufweist, die derart ausgebildet ist, dass der Spannstab mit für die räumliche Führung des Klemmhalters ausreichendem Bewegungsspielraum in der Durchgangsöffnung durch diese hindurchgesetzt ist. Mit der erfindungsgemäßen Lösung wird eine Reihe von Vorteilen erzielt. Durch die dreidimensionale Bewegungsführung und Verstellbarkeit des Klemmhalters wird die Einstellung und die Verbindung des Fixationselements mit Fixationsstangen besonders vereinfacht und erleichtert. Zudem trägt dies in besonderem Maße dazu bei, dass Stange und Grundkörper bzw. Klemmlager im Zustand der Kraftschlussverbindung nicht mit Kräften quer zu dem Spannstab verspannt werden. Dies erhöht die Festigkeit und Stabilität der Einrichtung in besonderem Maß. Hinsichtlich einfacher Bauform ist es besonders vorteilhaft, dass der Klemmhalter für die Klemmstange ein Lager bildet, das als solches in Gänze dreidimensional, also in alle Raumrichtungen bewegbar ist. Die Spannstabverbindung baut besonders einfach, da zu klemmende Teile einschließlich des an dem Grundkörper in alle Raumrichtungen geführten und verstellbaren Klemmhalters durch ein und denselben Zugstab der Spannverbindung gelöst und in steifer Verbindung aneinander befestigt werden. Spannkraft wird einheitlich für unterschiedliche Teile des Spannverbundes aufgebracht, nämlich sowohl für das Festsetzten der Fixationsstange in ihrem Klemmhalter, als auch zum Festsetzen des räumlich winkelverstellbaren Klemmhalters in einem Lagerelement an dem Grundkörper. Die erfindungsgemäße Verbindungs- und Bauform erlaubt es, die Bauteile relativ einfach auszuführen. Die Herstellungskosten der Einzelteile bleiben niedrig. Gegenüber herkömmlichen Einrichtungen wird der Aufwand für Einzelteile hinsichtlich Zahl, Handhabung und Nutzung erheblich reduziert. Insbesondere können durch verspanende Formgebung herzustellende Teile vermieden und statt dessen Leichtbauteile vorzugsweise aus Kunststoff-Spritzgussteilen eingesetzt werden.

Ein besonders universell verwendbares erfindungsgemäßes Fixationselement erreicht man dadurch, dass auf dem Spannstab wenigstens ein Grundkörper und am Grundkörper gegenüberliegend zwei Stangen-Klemmhalter mit ihren Lagerteilen in Reihe aufgesetzt sind. Zweckmäßig wird bei dieser Gestaltung der Grundkörper durch ein kreuzförmiges Element gebildet, umfassend einen zylindrischen Längskörperteil, der an seinen Enden Lagerflächen jeweils für einen Klemmhalter aufweist, und einen Querkörperteil, der wenigstens einen Knochennagel aufnimmt. Diese Formgebung des Grundkörpers, dessen Längskörperteil von dem Spannstab durchgriffen wird, weist für Druck- und/ oder Biegebelastung relativ große Widerstands-Querschnitte auf, so dass die Spannverbindung besonders fest und stabil ist. Zweckmäßig ist längs der Erstreckung des Querkörperteils in diesem ein Durchgangsschlitz ausgebildet, der Knochennägel in von dem Längskörperteil abstehenden Bereichen des Querkörperteils aufnimmt. Dies führt sowohl zu einer räumlich besonders günstigen Anordnung der Knochennägel, als auch zu einer besonders wirksamen und ausgeprägten Spannkraftübertragung längs des Längskörperteils. Damit erreicht man auch, dass ein Anziehen zum Spannen der Verbindung mit nur relativ geringem Anziehdrehmoment verbunden ist.

Aufgrund der erfindungsgemäßen Maßnahmen lässt sich ein Fixationselement, umfassend den Grundkörper und wenigstens einen mittels der Spannstabverbindung daran gehaltenen Stangen-/Klemmhalter, als kompaktes, in einfachem Verbund zusammengefügtes Teil für sich besonders einfach handhaben und auf zweckmäßig zwei Knochennägel aufsetzen.

Vorteilhaft wird die Spannstab-Verbindung durch eine Schrauben-Schaftverbindung gebildet. Diese weist zweckmäßig zwei Schrauben mit Längschaft auf, die in einem Durchgang des Grundkörpers aneinander geschraubt werden, so dass schon durch wenige Umdrehungen der beiden Schrauben relativ zueinander ein durch Verkürzung wirksamer Spannstab gebildet ist. Die damit verklemmten Teile werden in gerader Linie gleichmäßig auf Klemmdruck beansprucht. Statt dessen kann zum Beispiel auch ein durchgehender Spannstab Verwendung finden, der an wenigstens einem Ende in Schraubverbindung mit einer anziehbaren Mutter abschließt.

Eine besondere Gestaltung der Erfindung besteht darin, dass die Spannstabverbindung zusätzlich zu ihrer Funktion zum Festsetzen des Stangenhalters und der Stange ein Spannmittel bildet, das in dem Freigabe-Verbindungszustand wenigstens einen Knochennagel bewegbar in einen Grundkörper eingreifen lässt, der einteilig vorzugsweise aus Leichtwerkstoff wie Kunststoff gefertigt ist, während es den Knochennagel in dem Kraftschluss-Verbindungszustand kraftschlüssig stabil an dem Grundkörper festsetzt.

Vorteilhaft ist in dem Grundkörper wenigstens ein Schlitz zum verstellbaren Aufnehmen und zum Befestigen eines Knochennagels durch Klemmung ausgebildet, wobei der Grundkörper beim Spannen der Spannstabverbindung im Bereich des Schlitzes elastisch verformt wird. Zweckmäßig werden der Schlitz des Grundkörpers und wenigstens ein von ihm aufgenommener Knochennagel so dimensioniert, dass der Knochennagel im Freigabezustand der Spannstabverbindung zum Einstellen der Verbindungsposition zwischen Knochennagel und Grundkörper im Klemmschiebesitz von dem Schlitz aufgenommen wird. In dieser Ausführung lässt sich der Grundkörper besonders einfach und sicher auf Knochennägeln anbringen.

Erfindungsgemäß kann der Stangen-Klemmhalter vorteilhaft als selbständiges Klemmelement ausgebildet werden, das im Freigabezustand der Spannstabverbindung wenigstens eine Fixationsstange im Klemmsitz aufnimmt. Besonders vorteilhaft kann der Klemmhalter als selbsttätig wirkendes Klemmelement ausgebildet werden, das die Fixationsstange im materialelastischem Klemmsitz aufnimmt. Die Fixationsstange lässt sich wenigstens in axialer Richtung gegen Haftreibung zum Positionieren bewegen. Auch kann der Stangen-Klemmhalter zweckmäßig so ausgebildet werden, dass die Aufnahme für die Fixationsstange eine Öffnung aufweist, durch die die Stange im Freigabezustand der Spannstabverbindung in einen durch wenigstens ein verdrängbares Rastelement hergestellten Sitz gelangt. Dies erleichtert die Handhabung wesentlich, da der Fixationsstab aufgrund dieser Maßnahme durch eine Klick-Verbindung in eine Position zum Einstellen gesetzt wird. Ein Entweichen der Stange in Einsetzrichtung wird dadurch verhindert. Dennoch bleibt sie in wenigstens einer Dimension zum Einstellen bewegbar.

In weiterer besonders bevorzugter Gestaltung der Erfindung weist der dreidimensional bewegbare Stangen-Klemmhalter wenigstens zwei Durchgangsöffnungen auf, wobei eine erste Öffnung durch eine zentrale Durchgangs-Axialöffnung gebildet wird, durch die der Spannstab der Spannstabverbindung unter ausreichendem Belassen von radialem Spiel für die dreidimensionale Bewegung hindwchgesetzt ist, und wobei wenigstens eine zweite Öffnung quer zur Axialöffnung und ohne diese zu schneiden durch den Halter hindurchgeht und die Klemmaufnahme für die Fixationsstange bildet.

Zweckmäßig weist der an dem Grundkörper dreidimensional geführte Halter Kugelflächenabschitte auf, mit denen er im Formschluss zwischen auf dem Spannstab der Spannstabverbindung aufgereihten ringförmigen Lagerelementen gelagert ist, wobei das eine Lagerelement an dem auf dem Spannstab gehaltenen Grundkörper angeordnet ist. Eine besonders vorteilhafte Maßnahme besteht darin, dass die zentrale Durchgangsöffnung des Klemmhalters im Bereich wenigstens einer Lager-Kugelfläche aufgeweitet wird, um einen dadurch begrenzten sphärischen Halter-Verstellbereich vorzusehen. Um die Bauteilzahl besonders gering zu halten, wird der Klemmhalter zweckmäßig durch eine einteilige Kugel gebildet. Vorteilhaft wird ein ein solcher Halter mit einer Stangen-Klemmaufnahme ausgebildet, die an der Halteroberfläche einen offenen Schlitz zur Aufnahme der Fixationsstange bildet.

Um die Kraftschlussverbindung besonders zu verstärken, besteht eine erfindungsgemäße Maßnahme darin, dass wenigstens ein Teil der Klemmflächen der Fixationseinrichtung strukturiert ist. Zweckmäßig weist die Strukturierung eine Aufrauung, Rillen, Noppen und/oder ein entsprechendes Mittel zum Herstellen eines besonders innigen Eingriffs auf

Durch die erfindungsgemäßen Maßnahmen erreicht man eine relativ geringe Zahl von Bauteilen, die sich insbesondere als kostengünstige Leichtbauteile in Form eines kompakten Bausatzes in steriler Verpackung bereithalten lassen.

Weitere bevorzugte Merkmale, Ausführungsformen und Möglichkeiten der Erfindung gehen aus Unteransprüchen und der Beschreibung hervor. Besonders bevorzugte Ausführungsbeispiele werden anhand der Zeichnung näher erläutert. In der Zeichnung zeigen
- Fig. 1 und 2: in axonometrischer Darstellung und in Explosionsdarstellung eine erfindungsgemäße Fixationseinrichtung mit zwei durch zwei Fixationsstangen verbundenen Fixationselementen,
- Fig. 3: in axonometrischer Darstellung eine erfindungsgemäße Fixations- einrichtung mit zwei durch nur eine Fixationsstange verbundenen Fixationselementen und
- Fig. 4 und 5: eine axonometrischer Darstellung sowie in Explosionsdarstellung eine erfindungsgemäße Fixationseinrichtung mit zwei Verbin- dungsstangen und zwei Fixationselementen, die Knochennägel in unterschiedlicher Anordnung aufnehmen.

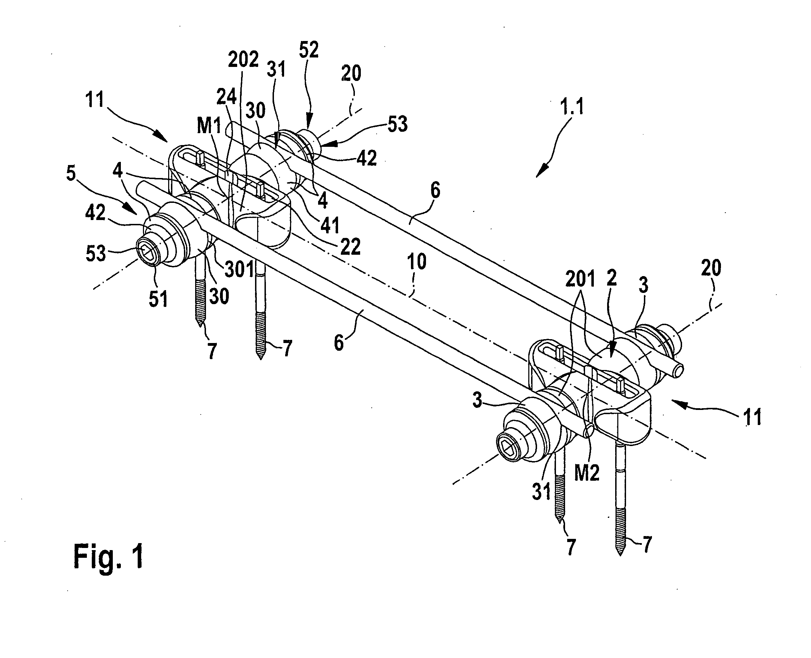

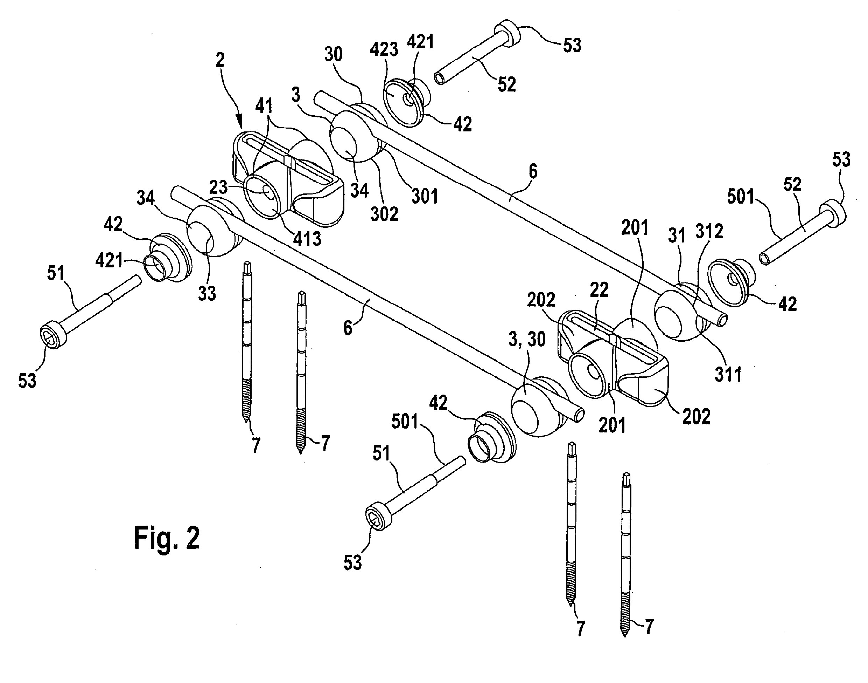

Aus Fig. 1 und 2 ist eine erste Ausführungsform einer erfindungsgemäßen Fixationseinrichtung 1 ersichtlich. Diese wird aus zwei erfindungsgemäßen Fixationselementen 11, Knochennägeln 7, an die die Elemente 11 angelegt sind, und zwei Fixationsstangen 6 gebildet. Es handelt sich um einen Fixateur, der mit den beiden Fixationsstangen 6 ein Knochengelenk, z.B. ein Handgelenk außen überbrückt, um eine Fixationsverbindung zwischen gebrochenen Knochenteilen herzustellen. Die Fixationselemente 11 sind übereinstimmend ausgebildet. Die Fixationsstangen 6 sind in Fig. 1 und 2 in paralleler Ausrichtung dargestellt. Die erfindungsgemäße Einrichtung erlaubt es aber, dass sie auch mit großer Abweichung von der Parallelausrichtung eingestellt werden können. Die Knochennägel 7 für das Fixationselement 11 stehen jeweils in einer Linie, die zumindest nahezu parallel zu den Fixationsstangen 6 verläuft.

Das Fixationslement 11 weist einen kreuzförmigen Grundkörper 2 auf, der einteilig und einstückig durch ein im Spritzgussverfahren geformtes Kunststoffteil hergestellt wird und in gewissem Maß zum Herstellen von Klemmverbindungen gegen materialelastische Rückstellkraft eigenverformbar ist. Zur Spritzgussfertigung wird ein hochfester Kunststoff relativ geringen spezifischen Gewichts verwendet. Ein solcher Körper lässt sich mit hoher Präzision in Form- und Maßhaltigkeit herstellen.

Der eine symmetrische Kreuzform aufweisende Grundkörper 2 ist durch einen kreiszylindrischen Längskörperteil 201 und einen Querkörperteil 202 gebildet, der im Ganzen ovalförmige Form aufweist und mit seinen Enden von dem Zylinderkörperteil 201 senkrecht absteht. Die Dimensionen der jeweils zu ihrer langen Erstreckungsrichtung senkrechten Querschnitte der Teile 201 und 202 liegen in der gleichen Größenordnung. In den Querkörperteil 202 ist ein randseitig geschlossener, flacher Durchgangsschlitz zum Bilden einer Aufnahme 22 für ein Paar Knochennägel 7 ausgebildet. Die Schlitzaufnahme 22 erstreckt sich mit dem Querkörperteil 202, wobei der Schlitz mit seiner flachen Fläche senkrecht zur zentralen Längsachse 20 des Längskörperteils 201 steht und an freien Schmalseiten des Querkörperteils 202 zum Aufsetzen auf die Knochennägel 7 offen ist. Der Längskörperteil 201 weist eine axiale Durchgangsbohrung 23 auf, durch die ein Spannstab 501 einer Spannverbindung 5 hindurchgesetzt ist.

Das Fixationselement 11 wird im Ganzen durch eine unverlierbare Reihenanordnung des Grundkörpers 2, von zwei Stangen-Klemmhaltern 3 und von Klemmhalter-Lagerelementen 41, 42 eines Lagers 4 auf dem Spannstab 501 gebildet. Die Stangen-Klemmhalter 3 schließen den Grundkörper 2 zwischen sich ein, und diese Anordnung wird mit zwei Lagen-ingelementen 42 in Form von kalottenartigen Kappen zwischen deren konkaven Ringseiten formschlüssig eingefasst.

Die beiden Stangen-Klemmhalter 3 sind übereinstimmend ausgebildet. Der Klemmhalter 3 wird durch eine einteilige, einstückige Klemmkugel 30 gebildet, die eine axiale zentrale Durchgangsöffnung 33 in Form einer Bohrung aufweist. Die Durchgangsöffnung 33 geht an ihren Öffnungsenden jeweils in eine konkave Ringausnehmung 34 über. Jede Kugel 30, die aus hochfestem Kunststoff gefertigt wird, wird formschlüssig von dem Lager 4 aufgenommen. Jedes Lager 4 weist ein jeweils am Ende des Längskörperteils 201 ausgeformtes Lagerelement 41 in Form einer.kalottenartigen, einen konkaven Lagerring bildenden Ausnehmung 413 und ein zugehöriges Lagerelement 42 auf. Jedes Lagerelement 42 weist ein Durchgangsloch 421 auf, mit dem es am Ende des Spannstabes 501 auf diesen aufgesteckt ist. Das Lagerelement 42 ist an seiner der Lagerkugel 30 zugewandten Seite mit einer konkaven Kreisringausnehmung 423 ausgebildet, die der konkaven Kreisringausnehmung 413 des Lagerelements 41 entspricht.

Die Klemmhalter-Lagerkugel 30 ist weiterhin mit einer Schlitzaufnahme 31 zum Aufnehmen und Halten der Fixationsstange 6 ausgebildet. Die Schlitzaufnahme 31 erstreckt sich in einem Kugel-Sekantenbereich in der Kugel-Äquatorialebene, ohne dass sie die axiale Durchgangsöffnung 33 der Kugel 30 schneidet. Die Schlitzaufnahme 31 ist an ihrem Boden entsprechend dem Kreisquerschnitt der Fixationsstange 6 aufgeweitet, so dass ein Sitz 311, den die Fixationsstange 6 an ihrem Ende durchfasst, gebildet ist. Im Übrigen bilden die Wände des Schlitzes 31 Rastelemente 312, die gegen materialelastische Rückstellkraft aufspreizbar sind, um die Fixationsstange 6 klemmend in den Sitz 311 einrasten zu lassen. Die Aufnahmequerschnitte der Schlitzaufnahme 31 und die Materialelastizität der Lagerkugel 30 werden so gewählt, dass die Stange 6 in eingerasteter Position durch Klemmung selbsttätig gehalten wird, wobei sie zum Positionieren und Justieren gegen Klemm-Reibkraft axial und rotatorisch bewegbar ist. Zudem ist die Lagerkugel 30 in ihrer Äquatorialebene durch einen Spalt 301 in zwei Hälften getrennt, die nur an der der Schlitzaufnahme 31 gegenüberliegenden Kugelseite einstückig durch einen Wandsteg 302 zusammengehalten werden. Der Spalt 301 wird durch einen Einschnitt gebildet. Die einteilige Lagerkugel 30 lässt sich besonders einfach handhaben. Statt dessen ist es auch möglich, die Kugelhälften vollständig getrennt voneinander auszubilden.

Die Durchgangsöffnung 33 geht durch den Spalt 302 hindurch. Dadurch erreicht man, dass die Kugelhälften mit gleichmäßiger und relativ geringer Spannkraft der Spannstabverbindung 5 zum Festsetzen der Stange 6 in der Schlitzaufnahme 31 gegeneinander gespannt werden. Die dabei über die Lagerelemente 41, 42 ausgeübte Spannkraft klemmt zudem die Lagerkugel 30 zwischen den Lagerelementen 41, 42 ein, so dass zugleich auch die Kugel 30 in ihrer Position einfach und sohr stabil fixiert wird. Der Durchmesser der Lagerkugel 30 ist nur geringfügig größer als der Querschnittsdurchmesser des zylindrischen Längskörperteils 203 und des Lagerelements 42. Infolgedessen bilden die Lagerkugeln 30 hochwirksame Klemmelemente in der auf dem Spannstab 501 aufgereihten Anordnung, wenn diese durch den Spannstab 501 aneinander gezogen werden.

Der Spannstab 501 der Spannverbindung 5 eines jeden Fixationselements 11 wird durch an ihren Fußenden axial aneinander schraubbare Schaftschrauben 51 und 52 gebildet. Zu diesem Zweck ist die Schaftschraube 51 an ihrem Fußende mit Außengewinde und die Schaftschraube 52 an ihrem Fußende mit Innengewinde versehen. Die Schaßschrauben 51, 52 weisen Köpfe 53 mit jeweils einer Innen-Mehrkantausnehmung zum Ansetzen eines Schraubendrehers auf. An den Lagerelementen 42 sind jeweils auf der der Lagerkugel 30 abgewandten Seite Ausnehmungen ausgeformt, in die die Schraubenköpfe 53 zum Gegeneinanderspannen der auf dem Spannstab 501 aufgereihten Teile eingreifen.

Die Lagerkugel 30 eines jeden Klemmhalters 3 wird so zwischen den Lagerringen 41, 42 formschlüssig gelagert, dass sie in ihrem Sitz in den Lagerelementen 41, 42 geführt und dreidimensional verstellbar ist, wenn die Spannverbindung 5 ausreichend gelöst ist Um einen ausreichend großen und begrenzten sphärischen Verstellbereich zu erhalten, ist die axiale Durchgangsöffnung 33 ausreichend groß bemessen, und die konkaven Ringausnehmungen 34 sind kegelartig von der Öffnung 33 her nach außen aufgeweitet.

Vorteilhaft und zweckmäßig wird der Verstellbereich so groß gewählt, dass, ausgehend von der in Fig. 1 dargestellten Position, in der die beiden Grundkörper 2 mit den Achsen 20 ihrer Längskörperteile 201 parallel in einer Ebene ausgerichtet sind, die Längskörperteile 201 bzw. deren Achsen 20 bei sich kreuzenden Stangen 6 gegeneinander um wenigstens 90° um eine die Mittelpunkte M1 und M2 der Kreuzstücke aufweisende gedachte Einrichtungsachse 10 torsionsartig verdreht bzw. verschwenkt werden können. Die beiden Achsen 20 lassen sich also mindestens in eine zueinander senkrechte Position bringen. Dabei liegt die gedachte Achse 10 nicht fest, sondern sie kann aus der in Fig. 1 dargestellten Grundposition in alle Raumrichtungen bewegt werden.

Der Schlitz 22 eines jeden Grundkörpers 2 und die von ihm im Paar aufgenommenen gleichen Knochennägel 7 sind so dimensioniert, dass die Knochennägel 7 auch bei vollständig gelöster Spannverbindung 5 im Schiebesitz gegen elastische Rückstellkraft des Wandmaterials des Grundkörpers 2 klemmend aufgenommen werden. An dem Schlitz 22 ist in der Mitte des Grundkörper-Kreuzstückes eine senkrecht zur axialen Durchgangsbohrung 23 gerichtete Ausnehmung 24 mit einer der Mehrkantausnehmung des Schraubenkopfes 53 entsprechenden Ausformung ausgebildet. Durch Einsetzen und Drehen des Drehwerkzeugs, mit dem auch die Spannverbindung 5 gespannt wird, lässt sich der Schlitz 22 gegen materialelastische Rückstellkraft der Schlitzwandung etwas aufweiten, um das Fixationselement 11 bequem auf das Knochennägel-Paar aufzusetzen. Dabei kommen die beiden Knochennägel 7 jeweils in dem Schlitzbereich zu liegen, der sich in den von dem Längskörperteil 201 abstehenden Enden des Querkörperteils 202 erstreckt. Die Anordnung der Knochennägel 7 in den Enden des Querkörperteils 202, die von dem Längskörperteil 201 abstehen, führt zu besonders wirksamen Klemmungen in dem erfindungsgemäßen Spannverbund. So erreicht man, dass der mittlere Bereich des Aufnahmeschlitzes 22 zwischen den Nägeln 7 in Richtung des Spannstabes 501 beim Spannen gleichmäßig und ausgeprägt elastisch druckverformbar ist. Auch die Ausformung der Teile des Grundkörper-Kreuzstückes in Paarung mit den verwendeten Kunststoffmaterialien bildet optimal wirksame Querschnitte zum dauerhaften und stabilen Spannen der Spannstabverbindung 5.

Die Operationstechnik zum Anlegen der erfindungsgemäßen Fixationeinrichtung erweist sich als besonders einfach und sicher. Nachdem zwei Paar Knochennägel 7 in im Wesentlichen einer Linie in üblicher Weise mit Eindrehwerkzeug und Bohrlehre gesetzt worden sind, wird auf jedes Nägelpaar jeweils das Fixationselement 11 aufgeschoben. Ein Drehwerkzeug wird in die Ausnehmung 24 gesteckt und in eine Position gedreht, dass der Schlitz 31 zum Aufschieben aufgeweitet wird. In zurückgestellt,er Drehposition des Werkzeugs wird dieses entnommen, und das Fixationselement sitzt zum Justieren durch Verstellen seiner Position ausreichend fest, aber verschiebbar auf den Knochennägeln 7. Nachdem beide Fixationselemente 11 an die zugehörigen Knochennägel 7 angelegt worden sind, werden die beiden Fixationsstangen 6 eingesetzt. Die Lagerkugeln 30 sind mit ihren Schlitzausnehmungen 31 an der Seite offen, die den zu verbindenden Knochenfeilen abgewandt ist, und sie lassen sich zum Einsetzen und Verstellen der Stangen 6 in festsetzbare Positionen bequem dreidimensional räumlich verstellen. Dabei gelangen die Stangen 6 zunächst mit ihren Enden in unverlierbare Klick-Rastpostionen, in denen sie zum Justieren dadurch verstellbar bleiben, dass sie einerseits durch die selbsttätige Klemmwirkung der klemmelastischen Kugel 30 axial und rotatorisch und andererseits durch die sphärische Verstellung der Kugel 30 in dem Lager 4 verlagerbar sind. Mit der erfindungsgemäßen in einer Einheit zusammengefassten universellen Verstellmöglichkeit zum Justieren wird erreicht, dass die beiden Fixationsstangen 6 zueinander weitgehend spannungsfrei gehalten werden. Dabei ist es sehr vorteilhaft, dass sowohl die Knochennägel 7 als auch die Fixationsstangen 6 durch die selbsttätige Klemmwirkung in der Schlitzaufnahme 22 des Grundkörpers 2 und in der Schlitzaufnahme 31 der Lagerkugel 30 ohne Einfluss der Spannverbindung 5 zum Justieren verstellbar sind. Zudem ist es aber auch möglich, die Spannstabverbindungen 5 geringfügig anzuziehen, um den Eigenklemmungen eine zusätzliche Komponente zum Justieren zu überlagern. Zum vollständigen Festsetzen der Teile aneinander wird die Spannstabverbindung 5 durch Festziehen und Spannen des Spannstabes 501 bis zu einem definierten Drehmoment gespannt. Dies gelingt erfindungsgemäß dadurch, dass sämtliche Klemmungen am Fixationselement 11 mit nur der einen Spannstabverbindung 5 einheitlich verwirklicht werden.

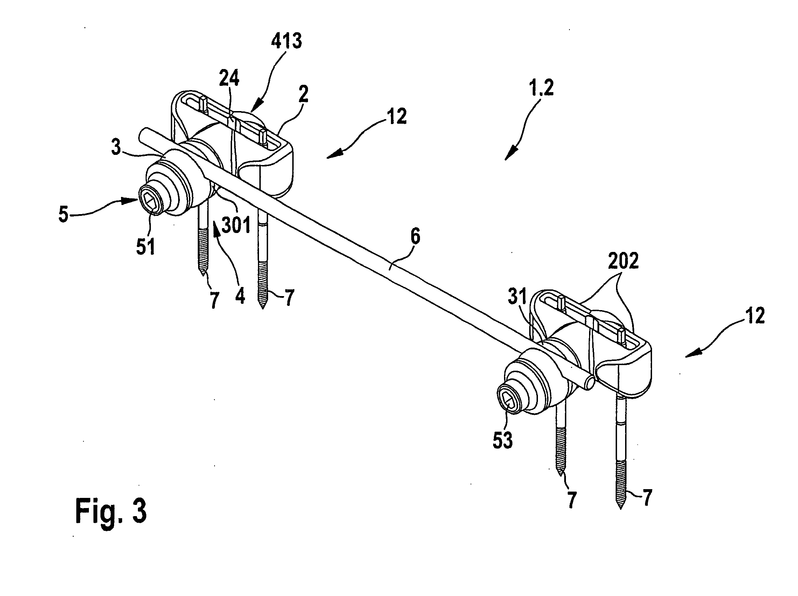

Aus Fig. 3 ist eine erfindungsgemäße Fixationseinrichtung 1.2 ersichtlich, die aus gleichen Bauteilen wie die Fixationseinrichtung 1.1 der Fig. 1 und 2 zusammengefügt ist Fixationselemente 12 dieser Anordnung werden dadurch gebildet, dass auf den Spannstab der Grundkörper 2 und der Stangen-Klemmhalter 3 nur an einem Ende des zylindrischen Längskörpers 201 aufgesetzt sind. An der anderen Seite dient die Ausnehmung 413 des Lagerelements 41 dazu, den Schraubenkopf 53 aufzunehmen. Zweckmäßig wird ein Spannstab 501mit Schraubelementen 51, 52 verwendet, die eine Änderung der Spannlänge in weitem Bereich erlauben, um sowohl für die Einrichtung nach Fig. 1 und 2, als auch für die Einrichtung nach Fig. 3 verwendet zu werden.

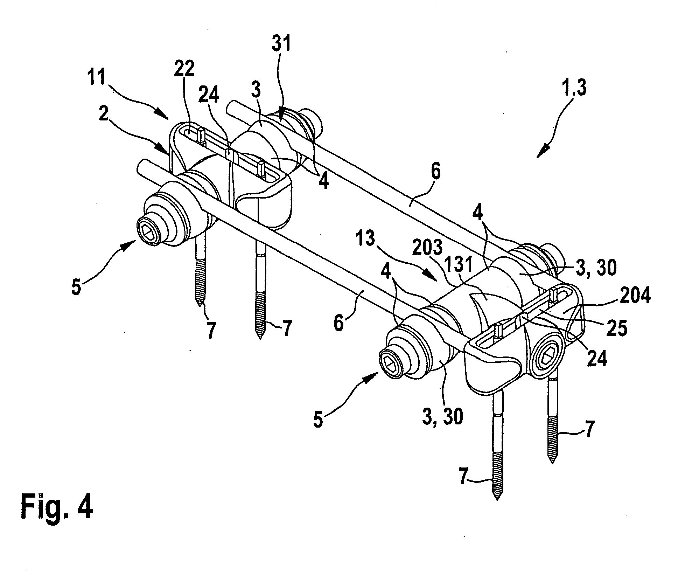

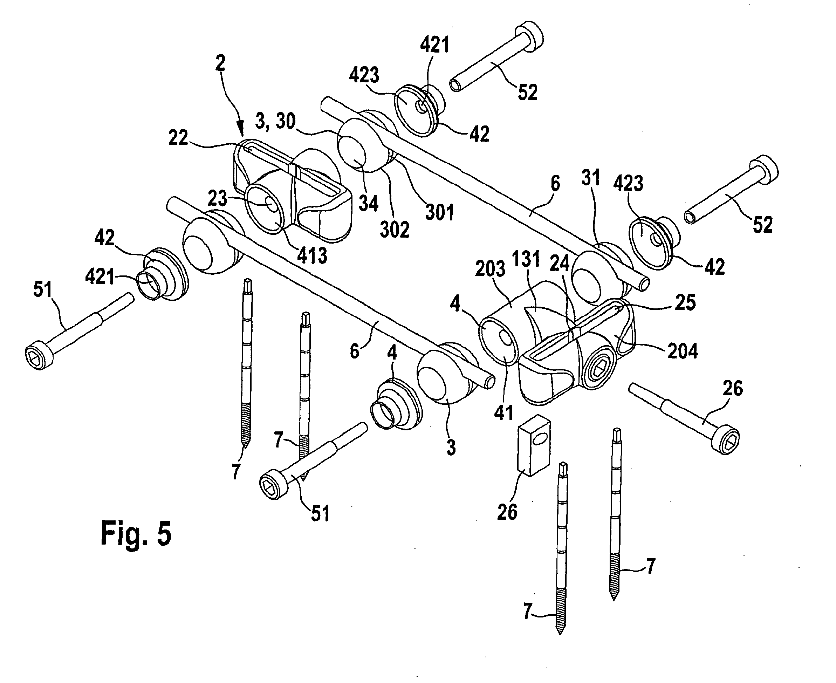

Fig. 4 und 5 zeigen eine erfindungsgemäße Fixationseinrichtung 1.3 in einer Ausführungsform mit einem modifizierten Fixationselement 13.

An einem Ende der Anordnung der im Wesentlichen sich parallel erstreckenden Fixationsstangen 6 ist das Fixationselement 11 angeordnet, wie es zu Fig. 1 und 2 beschrieben worden ist. Die Fixationsstangen 6 werden an ihren anderen Enden mit dem modifizierten Fixationselement 13 verbunden. Dieses ist wie das Fixationselement 11 ausgeführt mit der Ausnahme, dass anstelle des Längskörperteils 201 ein Längskörperteil 203 vorgesehen ist, der frei von einem Querteil bleibt und statt dessen mit einem zusätzlichen ovalförmigen Längskörperteil 204 versehen ist. Dieser erstreckt sich parallel zu dem Körperteil 203,mit dem er über ein Ansatzstück 131 verbunden ist. In dem Körperteil 204 ist in seiner Erstreckungsrichtung eine Schlitzaufnahme 25 für ein Paar Knochennägel 7 ausgebildet. Die Ausbildung dieses Schlitzes mit Werkzeug-Ausnehmung 24 entspricht der Ausbildung des Schlitzes in dem Teil 202 des anderen Fixationselements 11. Wie aus Fig. 4 und 5 ersichtlich, dient das Fixationselement 13 dazu, ein Paar von Knochennägeln 7 aufzunehmen, die quer, insbesondere wenigstens nahezu in einer zu den Stangen 6 senkrechten Linie stehen.

Im Ausführungsbeispiel der Fig. 4 und 5 ist der Körperteil 204 separat ausgebildet worden und über eine Schraubverbindung 26 mit dem am Körper 203 angeformten Ansatzstück 131 verbunden. Die Schraubverbindung 26 wird auch dazu genutzt, die Knochennägel 7 wie in der Schlitzaufnahme 22 in der Schlitzaufnahme 25 festzusetzen.

## Patentansprüche

1. Fixationseinrichtung (1) zum stabilen Verbinden wenigstens zweier Knochenteile eines gebrochenen Knochens, umfassend wenigstens einen Grundkörper (2) zur festen Aufnahme jeweils wenigstens eines Knochennagels (7), wenigstens eine Fixationsstange (6), die mittels Klemmverbindung mit dem Grundkörper (2) verbindbar ist, wobei Grundkörper (2) und Fixationsstange (6) in einem ersten Verbindungszustand der Fixationseinrichtung (1) zum Einstellen ihrer relativen Position freigegeben sind und in einem zweiten Verbindungszustand zum Fixieren der eingestellten Position durch Kraftschluss steif miteinander verbunden sind, einen an dem Grundkörper (2) winkelverstellbar gelagerten Klemmhalter (3), der die Fixationsstange (6) zum Verändern und Einstellen ihrer Position in einer Aufnahme (31) aufnimmt, ein den Klemmhalter (3) an dem Grundkörper (2) lagerndes Lager (4) und eine Verbindung (5) mit einem Spannstab (501), an dem zum Gegeneinanderspannen der Grundkörper (2), der Stangen-Klemmhatter (3) und das Lager (4) für den Klemmhalter (3) in Reihe angeordnet sind, wobei einerseits bei gelöster Spannstabverbindung (5) die Freigabe-Verbindung und andererseits bei ausreichend großer Schließspannung in der Spannstabverbindung (5) die stabile Kraftschluss-Verbindung hergestellt sind, wobei der Stangen-Klemmhalter (3) durch einen die Fixationsstange (6) sphärisch verstellenden Halter gebildet ist, der in einem Bereich zum Verstellen und Beibehalten der eingestellten Position an dem Grundkörper (2) in alle Raumrichtungen geführt ist, wobei sich der Klemmhalter (3) und wenigstens ein von dem Spannstab (501) durchfasstes Lagerelement (41, 42) des den Klemmhalter (3) mit dem Grundkörper (2) verbindenden Lagers (4) im die räumliche Führung herstellenden Formschluss befinden, **dadurch gekennzeichnet, dass** der Klemmhalter (3) auf dem Spannstab (501) aufgereiht ist, wobei er eine Durchgangsöffnung (33) aufweist, die derart ausgebildet ist, dass der Spannstab (501) mit für die räumliche Führung des Klemmhalters (3) ausreichendem Bewegungsspielraum in der Durchgangsöffnung (33) durch diese hindurchgesetzt ist.

2. Fixationseinrichtung nach Anspruch 1, **dadurch gekennzeichnet , dass** der Grundkörper (2) einteilig vorzugsweise aus einem Leichtwerkstoff, insbesondere Kunststoff, gebildet ist und dass jeweils wenigstens ein solcher Grundkörper (2), ein Stangen-Klemmhalter (3) und ein Klemmhalter-Lagerelement (41.42) in Reihe auf einen Spannstab (501) der Spannverbindung (5) aufgesetzt sind.

3. Fixationseinrichtung nach Anspruch 1 oder 2, **dadurch gekennzeich**n e t , dass auf den Spannstab (501) wenigstens ein Grundkörper (2) und am Grundkörper (2) gegenüberliegend zwei Stangen-Klemmhalter (3) mit ihren Lagerteilen (41, 42) in Reihe aufgesetzt sind.

4. Fixationseinrichtung nach einem der Ansprüche 1 bis 3, **dadurch gekennzeichnet, dass** die Spannstab-Verbindung (5) durch eine Schrauben-Schaftverbindung gebildet ist.

5. Fixationseinrichtung nach einem der Ansprüche 1 bis 4, **dadurch gekennzeichnet, dass** der Stangen-Klemmhalter (3) wenigstens zwei Durchgangsöffnungen (31, 33) aufweist, wobei eine erste Öffnung (33) durch eine zentrale Durchgangs-Axialöffnung gebildet ist, durch die der Spannstab (501) der Spannstabverbindung (5) unter ausreichendem Belassen von radialem Spiel für die dreidimensionale Bewegung hindurchgesetzt ist, und wobei wenigstens eine zweite Öffnung quer zur Axialöffnung (33) und ohne diese zu schneiden durch den Halter (3) hindurchgeht und die Klemmaufnahme (31) für die Fixationsstange (6) bildet.

6. Fixationseinrichtung nach Anspruch 5, **dadurch gekennzeichnet, dass** der Klemmhalter (3) Kugelflächenabschnitte aufweist, mit denen er im Formschluss zwischen auf dem Spannstab (501) der Spannstabverbindung (5) aufgereihten ringförmigen Lagerelementen (41, 42) gelagert ist, wobei das eine Lagerelement (41) an dem auf dem Spannstab (501) gehaltenen Grundkörper (2) vorzugsweise in einstückiger Ausbildung mit diesem angeordnet ist.

7. Fixationseinrichtung nach Anspruch 6, **dadurch gekennzeichnet, dass** die zentrale Durchgangsöffnung (33) des Halters (3) im Bereich wenigstens einer Lager-Kugelfläche aufgeweitet ist, um einen **dadurch** begrenzten sphärischen Halter-Verstellbereich vorzusehen.

8. Fixationseinrichtung nach Anspruch 6 oder 7, **dadurch gekennzeich**n e t, dass der Halter (3) durch eine einteilige oder zweiteilige Kugel (30) gebildet ist.

9. Fixationseinrichtung nach einem der Ansprüche 6 bis 9, **dadurch gekennzeichnet, dass** der Kugelflächen-Halter (3) einteilig und mit einer Klemmaufnahme (31) für die Fixationsstange (6) ausgebildet ist, wobei die Klemmaufnahme (31) zweckmäßig als an der Halteroberfläche offener Aufnahmeschlitz ausgebildet ist.

10. Fixationseinrichtung nach einem der Ansprüche 1 bis 9, **dadurch gekennzeichnet, dass** die Spannstabverbindung (5) zudem ein Spannmittel bildet, das in dem Freigabe-Verbindungszustand wenigstens einen Knochennagel (7) bewegbar in einen Grundkörper (2) eingreifen lässt, der einteilig vorzugsweise aus Leichtwerkstoff wie Kunststoff gefertigt ist, während es den Knochennagel (7) in dem Kraftschluss-Verbindungszustand kraftschlüssig stabil an dem Grundkörper (2) festsetzt.

11. Fixationseinrichtung nach Anspruch 10, **dadurch gekennzeichnet, dass** in dem Grundkörper (2) wenigstens ein Schlitz (22) zum verstellbaren Aufnehmen und zum Befestigen wenigstens eines Knochennagels (7) durch Klemmung ausgebildet ist, wobei der Grundkörpers (2) beim Spannen der Spannstabverbindung (5) im Bereich des Schlitzes (22) elastisch verformt wird.

12. Fixationseinrichtung nach Anspruch 11, **dadurch gekennzeichnet, dass** der Schlitz (22) des Grundkörpers (2) und wenigstens ein von ihm aufgenommener Knochennagel (7) so dimensioniert sind, dass der Knochennagel (7) im Freigabezustand der Spannstabverbindung (5) zum Einstellen der Verbindungsposition zwischen Knochennagel (7) und Grundkörper (2) im KlemmSchiebesitz von dem Schlitz (22) aufgenommen wird.

13. Fixationseinrichtung nach Anspruch 12, **dadurch gekennzeichnet, dass** der Schlitz (22) eine materialelastische Wandung derart aufweist, dass sich der Schlitzquerschnitt zum Einführen des Knochennagels (7) gegen Rückstellkraft mittels eines Werkzeugs erweitern lässt.

14. Fixationseinrichtung nach Anspruch 13, **dadurch gekennzeichnet, dass** am Schlitz (22) des Grundkörpers (2) eine Ausnehmung (24) zum Ansetzen des Werkzeugs für die elastische Schlitzaufweitung ausgebildet ist.

15. Fixationseinrichtung nach Anspruch 13 oder 14, **dadurch gekennzeichnet, dass** die Spannstabverbindung (5) mit einem Mittel (53) zum Spannen und Entspannen versehen ist, das sich mit dem Werkzeug für die Aufweitung des Grundkörper-Schlitzes (22) betätigen lässt.

16. Fixationseinrichtung nach einem der Ansprüche 1 bis 15, **dadurch gekennzeichnet, dass** im Freigabezustand der Spannstabverbindung (5) der Stangen-Klemmhalter (3) wenigstens eine Fixationsstange (6) in selbständig haltendem, zweckmäßig materialelastisch klemmendem Sitz aufnimmt, wobei sich die Fixationsstange (6) in diesem Sitz wenigstens in axialer Richtung zum Positionieren bewegen lässt.

17. Fixationseinrichtung nach einem der Ansprüche 1 bis 16, **dadurch gekennzeichnet, dass** an der Aufnahme (31) des Stangen-Klemmhalters (3) eine Öffnung ausgebildet ist, durch die die Fixationsstange (6) im Freigabezustand der Spannstabverbindung (5) in einen durch wenigstens ein verdrängbares Rastelement hergestellten Sitz gelangt.

18. Fixationseinrichtung nach einem der Ansprüche 1 bis 17, **dadurch gekennzeichnet, dass** wenigstens ein Grundkörper (2) mit wenigstens einer Aufnahme (21) ausgestattet ist, die wenigstens zwei Knochennägel (7) in Reihenanordnung längs der Erstreckungsrichtung einer Fixationsstange (6) aufnimmt.

19. Fixationseinrichtung nach einem der Ansprüche 1 bis 18, **dadurch gekennzeichnet, dass** wenigstens ein Grundkörper (203, 204) mit wenigstens einer Aufnahme (25) ausgestattet ist, die wenigstens zwei Knochennägel (7) in Reihenanordnung quer zur Erstreckungsrichtung einer Fixationsstange (6) aufnimmt.

20. Fixationseinrichtung nach einem der Ansprüche 1 bis 19, **dadurch gekennzeichnet, dass** wenigstens ein Teil der Klemmflächen der Fixationseinrichtung (1) zum Verstärken der Kraftschlussverbindung mit einer Strukturierung versehen ist, die zweckmäßig durch Aufrauung, Rillen; Noppen od. dgl. gebildet ist.

21. Fixationseinrichtung nach einem der Ansprüche 1 bis 20, **dadurch gekennzeichnet, dass** der Grundkörper (2) durch ein kreuzförmiges Element gebildet ist mit einem zylindrischen Längskörperteil (201), der an seinen Enden Lagerelement-Lagerflächen (413) jeweils für einen Klemmhalter (3) aufweist, und mit einem Querkörperteil (202), der wenigstens einen Knochennagel (7) aufnimmt, wobei zweckmäßig längs der Erstreckung des Querkörperteils (202) in diesem ein Durchgangsschlitz (31) ausgebildet ist, der Knochennägel (7) in von dem Längskörperteil (201) abstehenden Bereichen des Querkörperteils (202) aufnimmt.

22. Fixationseinrichtung nach Anspruch 21, **dadurch gekennzeichnet, dass** der Querkörperteil (202) ovalförmige Form mit gerundeten Flächen aufweist.

23. Fixationseinrichtung nach einem der Ansprüche 1 bis 22, **dadurch gekennzeichnet, dass** der Grundkörper (2) durch einen zylindrischen Längskörper (203) gebildet ist, der an seinen Enden Lagerflächen jeweils für einen Klemmhalter (3) aufweist, und dass an dem Längskörper (203) ein mit ihm steifer Aufnahmekörper (204) für wenigstens einen Knochennagel (7) angeordnet ist.

24. FixationsJement (11) gemäß Ausbildung in einer Einrichtung (1) nach einem der Ansprüche 1 bis 23, umfassend den Grundkörper (2) und wenigstens einen mittels der Spannstabverbindung (5) daran gehaltenen Stangen-Klemmhalter (3).

25. Bausatz in steriler Verpackung zum operationstechnischen Zusammenfügen einer Fixationseinrichtung, umfassend die Teile einer Fixationseinrichtung (1) nach einem der Ansprüche 1 bis 23.

## Claims

1. Fixation device (1) for the stable connection of at least two bone portions of a broken bone, including at least one base body (2) for tightly receiving at least one bone nail (7), at least one fixation rod (6) which can be connected to the base body (2) by means of a clamping joint, wherein base body (2) and fixation rod (6) are released in a first connection state of the fixation device (1) for adjusting their relative position and rigidly connected to each other by non-positive locking in a second connection state for fixing the adjusted position, a clamping holder (3) which is mounted with adjustable angle on the base body (2) and which receives the fixation rod (6) for altering and adjusting its position in a receptacle (31), a bearing (4) which mounts the clamping holder (3) on the base body (2), and a connection (5) to a tensioning rod (501) on which the base body (2), the rod clamping holder (3) and the bearing (4) for the clamping holder (3) are arranged in a row for tensioning relative to each other, wherein on the one hand the released connection is made when the tensioning rod connection (5) is released, and on the other hand the stable non-positive locking connection is made when there is sufficient closing tension in the tensioning rod connection (5), wherein the rod clamping holder (3) is formed by a holder which spherically displaces the fixation rod (6) and which is guided in all spatial directions in a region for displacing and maintaining the set position on the base body (2), the clamping holder (3) and at least one bearing element (41, 42) of the bearing (4) connecting the clamping holder (3) to the base body (2) being in a positive engagement which produces the spatial guiding, the tensioning rod (501) passing through the bearing element (41, 42), **characterised in that** the clamping holder (3) is lined up on the tensioning rod (501), comprising a through-opening (33) which is designed in such a way that the tensioning rod (501) passes through the through-opening (33) with sufficient play in the through-opening (33) for spatial guidance of the clamping holder (3).

2. Fixation device according to claim 1, **characterised in that** the base body (2) is formed in one piece, preferably from a lightweight material, in particular plastic, and **in that** at least one such base body (2), a rod clamping holder (3) and a clamping holder bearing element (41, 42) each are placed in a row on a tensioning rod (501) of the tensioning connection (5).

3. Fixation device according to claim 1 or 2, **characterised in that** on the tensioning rod (501) are placed in a row at least one base body (2) and on the base body (2) opposite each other two rod clamping holders (3) with their bearing portions (41, 42).

4. Fixation device according to any one of claims 1 to 3, **characterised in that** the tensioning rod connection (5) is formed by a screw/shank connection.

5. Fixation device according to any one of claims 1 to 4, **characterised in that** the rod clamping holder (3) comprises at least two through-openings (31, 33), wherein a first opening (33) is formed by a central axial through-opening through which the tensioning rod (501) of the tensioning rod connection (5) is passed, leaving sufficient radial play for the three-dimensional movement, and wherein at least one second opening passes through the holder (3) transversely to the axial opening (33) and without intersecting the latter and forms the clamping receptacle (31) for the fixation rod (6).

6. Fixation device according to claim 5, **characterised in that** the clamping holder (3) comprises spherical surface sections with which it is mounted in positive engagement between annular bearing elements (41, 42) in a row on the tensioning rod (501) of the tensioning rod connection (5), wherein one bearing element (41) is arranged on the base body (2) which is held on the tensioning rod (501), preferably formed integrally with the base body (2).

7. Fixation device according to claim 6, **characterised in that** the central through-opening (33) of the holder (3) is expanded in the region of at least one spherical surface of the bearing in order to provide a spherical holder displacement region which is limited thereby.

8. Fixation device according to claim 6 or 7, **characterised in that** the holder (3) is formed by a one-part or two-part ball (30).

9. Fixation device according to any one of claims 6 to 8, **characterised in that** the spherical-surface holder (3) is in one piece and designed with a clamping receptacle (31) for the fixation rod (6), the clamping receptacle (31) being appropriately designed as an open receiving slot on the holder surface.

10. Fixation device according to any one of claims 1 to 9, **characterised in that** the tensioning rod connection (5) in addition forms a tensioning means which in the released connection state lets at least one bone nail (7) movably engage in a base body (2) which is manufactured in one piece preferably from lightweight material such as plastic, while it fixes the bone nail (7) to the base body (2) in a non-positive locking stable relationship in the non-positive locking connection state.

11. Fixation device according to claim 10, **characterised in that** in the base body (2) is formed at least one slot (22) for adjustably receiving and fastening at least one bone nail (7) by clamping, the base body (2) being elastically deformed in the region of the slot (22) upon tensioning the tensioning rod connection (5).

12. Fixation device according to claim 11, **characterised in that** the slot (22) in the base body (2) and at least one bone nail (7) received by it are dimensioned such that in the released state of the tensioning rod connection (5) the bone nail (7) is received by the slot (22) in a clamping/sliding fit for adjusting the connecting position between bone nail (7) and base body (2).

13. Fixation device according to claim 12, **characterised in that** the slot (22) has a wall of elastic material such that the slot cross-section can be expanded for introducing the bone nail (7) against return force by means of a tool.

14. Fixation device according to claim 13, **characterised in that** on the slot (22) in the base body (2) is formed a recess (24) for applying the tool for elastic expansion of the slot.

15. Fixation device according to claim 13 or 14, **characterised in that** the tensioning rod connection (5) is provided with a means (53) for tensioning and relaxing, which can be actuated with the tool for expansion of the slot (22) in the base body.

16. Fixation device according to any one of claims 1 to 15, **characterised in that**, in the released state of the tensioning rod connection (5), the rod clamping holder (3) receives at least one fixation rod (6) in a press fit which holds independently and appropriately clamps due to the elasticity of the material, wherein the fixation rod (6) in this fit is movable at least in the axial direction for positioning.

17. Fixation device according to any one of claims 1 to 16, **characterised in that** on the receptacle (31) of the rod clamping holder (3) is formed an opening through which the fixation rod (6) passes in the released state of the tensioning rod connection (5) into a fit produced by at least one displaceable latch element.

18. Fixation device according to any one of claims 1 to 17, **characterised in that** at least one base body (2) is provided with at least one receptacle (21) which receives at least two bone nails (7) arranged in a row along the direction in which a fixation rod (6) extends.

19. Fixation device according to any one of claims 1 to 18, **characterised in that** at least one base body (203, 204) is provided with at least one receptacle (25) which receives at least two bone nails (7) arranged in a row transversely to the direction in which a fixation rod (6) extends.

20. Fixation device according to any one of claims 1 to 19, **characterised in that** at least one portion of the clamping surfaces of the fixation device (1) is provided with structuring which is appropriately formed by roughening, grooves, knobs or the like, for strengthening the non-positive locking connection.

21. Fixation device according to any one of claims 1 to 20, **characterised in that** the base body (2) is formed by a cross-shaped element having a cylindrical longitudinal body portion (201) which comprises bearing surfaces (413) of bearing elements at its ends for a clamping holder (3) each, and having a transverse body portion (202) which receives at least one bone nail (7), along the extent of the transverse body portion (202) in the latter being appropriately formed a through-slot (31) which receives bone nails (7) in regions of the transverse body portion (202) projecting from the longitudinal body portion (201).

22. Fixation device according to claim 21, **characterised in that** the transverse body portion (202) has an oval shape with rounded surfaces.

23. Fixation device according to any one of claims 1 to 22, **characterised in that** the base body (2) is formed by a cylindrical longitudinal body (203) which at its ends comprises bearing surfaces for a clamping holder (3) each, and **in that** on the longitudinal body (203) is arranged a receiving body (204) rigidly connected to it for at least one bone nail (7).

24. Fixation element (11) as embodied in a device (1) according to any one of claims 1 to 23, including the base body (2) and at least one rod clamping holder (3) held thereon by means of the tensioning rod connection (5).

25. Kit in a sterile package for surgical assembly of a fixation device, including the parts of a fixation device (1) according to any one of claims 1 to 23.

## Revendications

1. Dispositif de fixation (1) permettant la liaison stable d'au moins deux parties d'un os brisé comprenant au moins un corps de base (2) conçu pour recevoir au moins une broche (7) et au moins une tige de fixation (6) qui peut être reliée au corps de base, au moyen d'une liaison par serrage, au moyen duquel le corps de base (2) et la tige de fixation (6) sont libérés dans un premier état de liaison du dispositif de fixation (1) pour régler leur position relative et sont reliés fixement l'un à l'autre dans un second état de liaison pour fixer la position réglée par adhérence, un support de serrage (3) monté sur le corps de base (2) dont l'angle peut être réglé, qui accueille la tige de fixation (6) pour modifier et régler sa position dans un logement (31), un palier (4) supportant le support de serrage (3) sur le corps de base et une liaison (5) avec une barre de serrage (501) sur laquelle, pour serrer l'un contre l'autre, le corps de base (2), le support de serrage-tiges (3) et le palier (4) pour le support de serrage (3) sont montés en série, au moyen desquels sont construites, d'un côté, la liaison-libération pour une liaison par barre de serrage (5) desserrée, et d'un autre côté, la liaison-adhérence stable en tant que sollicitation de fermeture suffisamment grande dans la liaison par barre de serrage (5), le support de serrage tiges (3) étant formé grâce à un support sphérique déplaçant la tige de fixation (6), qui est guidé dans toutes les directions dans l'espace dans une certaine plage pour régler et conserver sa position réglée sur le corps de base (2), le support de serrage (3) et au moins un élément (41, 42) de palier traversé par une barre de serrage (501) du palier (4) reliant le support de serrage (3) au corps de base (2) se trouvent dans une liaison de forme qui permet le guidage dans l'espace, **caractérisé en ce que** le support de serrage (3) est rangé sur la barre de serrage (501), au moyen duquel une ouverture (33) de passage, qui est constituée de telle façon que la tige de serrage (501) est positionnée en travers à l'intérieur pour le guidage dans l'espace du support de serrage (3) ménageant ainsi une liberté de mouvement dans l'ouverture (33) de passage à travers celle-ci.

2. Dispositif de fixation selon la revendication 1, **caractérisé en ce que** le corps de base (2) est constitué de préférence d'un seul tenant d'un matériau léger, en particulier en matière plastique, et **en ce que** au moins un tel corps de base (2), un support de serrage-tiges (3) et un élément palier-support de serrage (41, 42) sont disposés respectivement en série sur une tige de serrage (501) de la liaison de serrage (5).

3. Dispositif de fixation selon la revendication 1 ou la revendication 2, **caractérisé en ce que**, sur la tige de serrage (501) au moins un corps de base (2) et deux supports de serrage-tiges (3) se trouvant à l'opposé sur le corps de base (2) sont disposés en série avec leurs parties (41, 42) de palier.

4. Dispositif de fixation selon l'une des revendications 1 à 3, **caractérisé en ce que** la liaison par barre de serrage (5) est formée d'une liaison de la vis/tige.

5. Dispositif de fixation selon l'une des revendications 1 à 4, **caractérisé en ce que** le support de serrage-tiges (3) représente au moins deux ouvertures (31, 33) de passage, une première ouverture (33) étant constituée d'une ouverture axiale centrale de passage, par laquelle la tige de serrage (501) de la liaison par barre de serrage (5) est disposée en travers à l'intérieur en laissant une quantité suffisante de jeu radial pour le mouvement tridimensionnel, et forme au moins une deuxième ouverture traversant obliquement l'ouverture axiale (33) et sans couper celle-ci par l'intermédiaire du support (3) et l'élément de réception par serrage (31) pour la tige de fixation.

6. Dispositif de fixation selon la revendication 5, **caractérisé en ce que** le support de serrage (3) présente des sections de surfaces sphériques avec lesquelles il est conservé en liaison de forme entre les éléments (41, 42) de palier rangés sous forme d'anneaux sur la barre de serrage (501) de la liaison par barre de serrage (5), l'élément de palier (41) étant agencé sur le corps de base (2) maintenu sur la barre de serrage (501), de préférence formée d'une seule pièce avec celui-ci.

7. Dispositif de fixation selon la revendication 6, **caractérisé en ce que** l'ouverture centrale (33) du passage du support (3) est élargie dans la zone d'au-moins une surface sphérique de palier, afin de prévoir une zone de déplacement du support sphérique limitée.

8. Dispositif de fixation selon l'une des revendications 6 ou 7, **caractérisé en ce que** le support (3) est formé d'une sphère (30) en une partie ou en deux parties.

9. Dispositif de fixation selon l'une des revendications 6 à 9, **caractérisé en ce que** le support (3) à surface sphérique est formé d'un seul tenant et avec un élément de réception par serrage (31) pour la tige de fixation (6), l'élément de réception par serrage (31) étant formé judicieusement comme une fente de réception ouverte sur la surface supérieure du support.

10. Dispositif de fixation selon l'une des revendications 1 à 9, **caractérisé en ce que** la liaison (5) par barre de serrage forme de plus un moyen de serrage qui saisit dans l'état de liaison-libération au moins une broche (7) mobile dans un corps de base (2), qui est réalisé de préférence d'un seul tenant en matériau léger comme la matière plastique tandis qu'il maintient par adhérence la broche (7) dans l'état de liaison-adhérence de façon stable sur le corps de base (2).

11. Dispositif de fixation selon la revendication 10, **caractérisé en ce que** dans le corps de base (2) au moins une fente (22) est formée par serrage pour accueillir par réglage et pour fixer par serrage au moins une broche (7), le corps de base (2) étant formé de façon élastique lors du serrage de la liaison (5) par barre de serrage dans la zone de la fente (22).

12. Dispositif de fixation selon la revendication 11, **caractérisé en ce que** la fente (22) du corps de base (2) et au moins une broche (7) accueillie par lui sont dimensionnées de telle façon que la broche (7) est accueillie dans l'état libéré de la liaison (5) par barre de serrage pour régler la position de liaison entre la broche (7) et le corps de base (2) dans le siège coulissant de serrage à partir de la fente (22).

13. Dispositif de fixation selon la revendication 12, **caractérisé en ce que** la fente (22) présente une paroi en matériau élastique de telle façon que la section de la fente s'élargit pour guider la broche (7) contre une force inverse au moyen d'un outil.

14. Dispositif de fixation selon la revendication 13, **caractérisé en ce que** sur la fente (22) du corps de base (2), un évidement (24) est constitué pour permettre la mise en place de l'outil pour l'élargissement élastique de la fente.

15. Dispositif de fixation selon la revendication 13 ou la revendication 14, **caractérisé en ce que** la liaison (5) par barre de serrage est prévue avec un moyen (53) de fixation et de détente, qui est actionné à l'aide de l'outil pour élargir la fente (22) du corps de base.

16. Dispositif de fixation selon l'une des revendications 1 à 15, **caractérisé en ce que** dans l'état de libération de la liaison (5) par barre de serrage, le support de serrage-tiges (3) accueille au moins une tige de fixation (6) dans un logement de serrage constitué judicieusement en matériau élastique en position autonome, la tige de fixation (6) se déplaçant dans ce logement pour se positionner au moins en direction axiale.

17. Dispositif de fixation selon l'une des revendications 1 à 16, **caractérisé en ce qu'**une ouverture est constituée dans le logement (31) du support de serrage-tiges (3), par l'intermédiaire de laquelle la tige de fixation (6) parvient dans l'état de libération de la liaison (5) par barre de serrage dans un emplacement représenté grâce à au-moins un élément d'encliquetage compressible.

18. Dispositif de fixation selon l'une des revendications 1 à 17, **caractérisé en ce qu'**au moins un corps de base (2) est équipé d'un logement (21) qui accueille au moins deux broches (7) dans un montage en série dans le sens de la longueur de la direction d'étirement d'une tige de fixation (6).

19. Dispositif de fixation selon l'une des revendications 1 à 18, **caractérisé en ce qu'**au moins un corps de base (203, 204) est équipé d'au moins un logement (25), qui accueille au moins deux broches (7) dans un montage en série dans le sens transversal de la direction d'étirement d'une tige de fixation (6).

20. Dispositif de fixation selon l'une des revendications 1 à 19, **caractérisé en ce qu'**au moins une partie des surfaces de serrage de l'installation de fixation (1) est formée pour renforcer la liaison par adhérence avec une structure, qui est constituée judicieusement d'une surface rugueuse, de rainures, de boutons ou d'éléments similaires.

21. Dispositif de fixation selon l'une des revendications 1 à 20, **caractérisé en ce que** le corps de base (2) est formé autour d'un élément en forme de croix avec une partie de corps longitudinale (201) cylindrique qui présente à ses extrémités des surfaces de palier-éléments de palier (413) respectivement pour un support de serrage (3) et comprenant une partie de corps transversale (202), qui accueille au moins une broche (7), une fente de passage d'ouverture (31) étant formée dans celui-ci judicieusement dans le sens de la longueur de l'étirement de la partie de corps transversale (202), accueillant la broche (7) dans des zones en saillie de la partie de corps transversale (202) provenant de la partie de corps longitudinale (201).

22. Dispositif de fixation selon la revendication 21, **caractérisé en ce que** la partie de corps transversale (202) présente une forme ovale avec des surfaces arrondies.

23. Dispositif de fixation selon l'une des revendications 1 à 22, **caractérisé en ce que** le corps de base (2) est formé par un corps longitudinal (203) cylindrique, qui présente respectivement à ses extrémités des surfaces de palier pour un support de serrage (3) et qui, avec lui, est agencé sur le corps longitudinal (203) avec le corps de réception rigide (204) pour au moins une broche (7).

24. Élément de fixation (11), selon la formation dans un dispositif (1) selon l'une des revendications 1 à 23, comprenant le corps de base (2) et au moins un support de serrage-tiges (3) maintenu sur celui-ci au moyen de la liaison par barre de serrage (5).

25. Kit en emballage stérile pour des assemblages technico-opérationnels d'un dispositif de fixation, comprenant les pièces d'un dispositif de fixation (1) selon l'une des revendications 1 à 23.
